(19) Europäisches Patentamt
European Patent Office
Office européen des brevets

(11) **EP 2 000 122 A2**

(12) **EUROPEAN PATENT APPLICATION**

(43) Date of publication:
**10.12.2008 Bulletin 2008/50**

(51) Int Cl.:
*A61K 8/34* (2006.01)   *A61K 8/49* (2006.01)
*A61Q 17/00* (2006.01)   *A01N 43/80* (2006.01)

(21) Application number: **08160585.9**

(22) Date of filing: **11.07.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.07.2005  GB 0514219**
**11.07.2005  US 698038 P**

(62) Document number(s) of the earlier application(s) in accordance with Art. 76 EPC:
**06755759.5 / 1 906 734**

(71) Applicant: **Thor Specialities (UK) Limited Wincham, Northwich Cheshire CW9 6G B (GB)**

(72) Inventors:
• **Seal, Ken**
  **Wincham Northwich Cheshire, CW9 6GB (GB)**
• **Hahn, Peter Erich**
  **67346, Speyer (DE)**

(74) Representative: **Huhn, Michael**
**Isenbruck Bösl Hörschler Wichmann Huhn Patentanwälte**
**Theodor-Heuss-Anlage 12**
**68165 Mannheim (DE)**

Remarks:
This application was filed on 17-07-2008 as a divisional application to the application mentioned under INID code 62.

(54) **Microbiocidal composition**

(57)    A microbiocidal composition comprising:
a non-halogenated 2-alkyl-3-isothiazolone, and chlorphenesin and/or an alkane diol,
wherein the alkyl group in the non-halogenated 2-alkyl-3-isothiazolone comprises 1 to 4 carbon atoms, the alkane diol comprises from 5 to 12 carbon atoms, and the hydroxyl groups of the alkane diol are vicinally substituted.

EP 2 000 122 A2

**Description**

The present invention relates to microbiocidal compositions, in particular microbiocidal compositions for use in cosmetic formulations.

It is desirable for cosmetic formulations to have a long shelf life. However, cosmetic formulations tend to degrade over time owing to bacterial or fungal growth in the formulation. There is therefore a desire to produce formulations that have an improved resistance to microbial attack, both from bacteria and fungi.

While chlorinated isothiazolones have been used in cosmetic formulations and are effective in relatively small concentrations, they have been found to be unstable with variations in pH and/or temperature. The chlorinated isothiazolones may also trigger allergies and this further restricts their use. Moreover, they may be incompatible with some cosmetic raw materials, especially raw materials which act as nucleophiles or reducing agents.

It is an object of the present invention to overcome, or at least mitigate, some of the problems associated with the compositions of the prior art.

Accordingly, the present invention provides a microbiocidal composition comprising:

a non-halogenated 2-alkyl-3-isothiazolone, and
chlorphenesin and/or an alkane diol,
wherein the alkyl group in the non-halogenated 2-alkyl-3-isothiazolone comprises 1 to 4 carbon atoms,
the alkane diol comprises 5 to 12 carbon atoms, and
the hydroxyl groups of the alkane diol are vicinally substituted.

Non-halogenated 2-alkyl-3-isothiazolones may act as biocidal agents. However, when used alone their performance in preventing growth of bacteria and fungi is relatively poor in comparison with an equivalent concentration of chlorinated isothiazolones.

However, non-halogenated 2-alkyl-3-isothiazolones have unexpectedly been found to be relatively stable to variations in pH and/or temperature compared to chlorinated isothiazolones. Furthermore, non-halogenated 2-alkyl-3-isothiazolones have been found to be more compatible than chlorinated isothiazolones with the raw materials typically present in cosmetic formulations.

It has also surprisingly been found that the combination of the non-halogenated 2-alkyl-3-isothiazolone and the alkane diol results in a synergistic effect in preventing microbial growth. As a consequence, a lower concentration of the non-halogenated isothiazolone may be used to effectively prevent microbial growth.

It has also been surprisingly found that the combination of a non-halogenated 2-alkyl-3-isothiazolone and chlorphenesin has a synergistic effect in preventing microbial growth.

The combination of the 2-alkyl-3-isothiazolone, chlorphenesin and the alkane diol results in a particularly effective microbiocidal composition in which all the ingredients have been found to act synergistically, i.e. the biocidal effect is greater than would be expected purely on the efficacy of the components alone.

Preferably, the 2-alkyl-3-isothiazolone and the alkane diol are present in the composition in a ratio (wt.%) range of from 1:3 to 1:400, more preferably of from 1:10 to 1:50, still more preferably of from 1:15 to 1 to 30. The 2-alkyl-3-isothiazolone and the alkane diol may be present in the composition in a ratio (wt.%) range of from 1:25 to 1:48, still more preferably from 1:30 to 1:45.

Preferably, the 2-alkyl-3-isothiazolone and the chlorphenesin are present in the composition in a ratio (wt.%) range of from 10:3 to 1:400, more preferably of from 1:10 to 1:50, still more preferably from 1:15 to 1:45, more preferably 1:15 to 1:30.

The ratio of 2-alkyl-3-isothiazolone:alkane diol:chlorphenesin may be in the range of from 1:10:10 to 1:20:20 (i.e. varying independently the amounts of alkane diol and/or chlorphenesin between their preferred upper and lower limits relative to the amount of isothiazolone).

The alkyl group in the 2-alkyl-3-isothiazolone may be substituted or non-substituted and may be branched or linear. The alkyl group may be substituted with one or more of the following: hydroxyl, cyano, alkylamino, carboxy, carboalkoxy and alkylthio.

Preferably, the non-halogenated 2-alkyl-3-isothiazolone is 2-methyl-3-isothiazolone.

It has been found by the inventors that different alkane diols exhibit different biocidal efficacies in combination with 2-alkyl-3-isothiazolones.

The alkane diol may comprise 5 to 12 carbon atoms. The alkane diol may comprises 5 to 10 carbon atoms, more preferably 6 - 8 carbon atoms, which may be in a linear or branched chain, preferably a linear chain. Preferably the alkane diol comprises 6 or more carbons. The alkane diol may be selected from one or more of 1,2-pentanediol, 1,2-hexanediol and 1,2-octanediol. The alkane diol may be 1,2-octanediol. It has been found that the antimicrobial activity of C5 alkane diols is surprisingly poor, the reasons for which are not fully understood (see Table 1A of the examples). Preferably, therefore, the alkane diol comprises at least 6 carbons.

The alkane diol may comprises 9 to 12 carbons, preferably 10, 11 or 12 carbons, most preferably 10 carbons. The alkane diol may be selected from 1,2-decanediol and 1,2-dodecanediol. It has been surprisingly found that alkane diols comprising 9 to 12 carbons offer greater biocidal efficacy than lower alkane diols.

Preferably, the composition further comprises a water-soluble organic solvent. Preferably, the organic solvent comprises one or more of ethylene glycol, diethylene glycol, polyethylene glycol, propylene glycol, dipropylene glycol, polypropylene glycol, phenoxyethanol and benzyl alcohol. Phenoxyethanol and benzyl alcohol have been found to be advantageous as solvents, since they also in themselves act as microbiocidal agents..

The composition may further comprise water.

Preferably, the composition further comprises (i) propylene glycol and/or dipropylene glycol and (ii) water.

The composition may further comprise one or more additional microbial agents. Examples of the additional microbial agents include one or more of benzyl alcohol, 2,4-dichlorobenzyl alcohol, 2-phenoxyethanol, 2-phenoxyethanol hemiformal, phenylethyl alcohol, 5-bromo-5-nitro-1,3-dioxane, formaldehyde and formaldehyde-releasing substances, dimethylol dimethylhydantoin, glyoxal, glutaraldehyde, sorbic acid, benzoic acid, salicylic acid, d-hydroacetic acid, p-hydroxybenzoic acid ester, chloroacetamide, N-methylchloro acetamide, phenols (such as p-chloro-m-cresol and o-phenylphenol), N-methylurea, N,N'-dimethylurea, benzyl formal, 4,4-dimethyl-1,3-oxazolidine, 1,3,5-hexahydrotriazine, a quarternary ammonium compound (such as N-alkyl-N,N-dimethylbenzyl ammonium chloride and di-n-decyldimethyl ammonium chloride), cetyl pyridinium chloride, diguanidin, polyhexamethylenebiguanide, chlorhexidine, 1,2-dibromo-2,4-dicyanobutane, 3,5-dichloro-4-hydroxybenzaldehyde, ethylene glycol hemiformal, tetra-(hydroxymethyl)-phosphonium salts, dichlorophene, 2,2-dibromo-3-nitrilopropionic acid amide, methyl-N-benzimidazole-2-ylcarbamate, 2-n-octylisothiazolin-3-one, 4,5-trimethyl-2-methylisothiazolin-3-one, 4,5,dichloro-2-n-octyl-4-isothiazolin-3-one, 2,2'-dithiodibenzoic acid-di-N-methylamide, benzisothiazolone derivatives, 2-thiocyanomethylthiobenzothiazole, 2-(hydroxymethyl)aminoethanol, p-tolyldiiodomethylsulphone, 1-(3-chloroallyl)-3,5,7-triaza-1-azoniaadamantane chloride, and a C-formal (such as 2-hydroxymethyl-2-nitro-1,3-propandiol, 2-bromo-2-nitropropane-1,3-diol, and reaction products of hydantoin). Of these, 2-phenoxyethanol and benzylalcohol, either separately or in combination, are particularly preferred.

It will be appreciated that the microbiocidal composition of the present invention may contain other ingredients. For example, the composition may further comprise one or more ingredients selected from thickeners, anti-foaming agents, substances for adjusting pH values, aromas, dispersion agents and colouring agents.

The present invention further provides for the use of the microbiocidal composition as herein described in a cosmetic formulation and also a cosmetic formulation comprising the composition. The cosmetic formulation may be, for example, a moisturising cream, sunscreen, liquid soap, conditioner or shampoo.

The composition of the present invention may also be used in products such as paints, plaster compositions for the application to walls, lignin sulphonates, whitewashes, adhesives, photochemicals, products containing casein, products containing starch, asphalt emulsions, surfactant solutions, fuels, cleaning agents, water systems, polymer dispersions, and lubricants.

The microbiocidal composition according to the present invention may typically constitute 30 to 3000 parts per million of the total weight of the cosmetic formulation.

Preferably, the alkane diol is present in the composition (or in the product/cosmetic formulation) in a concentration of 500 ppm or above. At these concentrations, it has surprisingly been found that as little as 10 ppm of a 2-alkyl-3-isothiazolone can be used to prevent growth of *Pseudomonas aeruginosa* and *Staphylococcus aureus.*

Preferably, the alkane diol is present in the composition (or in the product/cosmetic formulation) in a concentration of 1000 ppm or above. At these concentrations, it has surprising been found that as little as 50 ppm of a 2-alkyl-3-isothiazolone can be used to prevent growth of *Aspergillus niger* and *Enterococcus faecalis.*

Preferably, the alkane diol is present in the composition (or in the product/cosmetic formulation) in a concentration of 2000 ppm or above, more preferably 2500 ppm or above. At these concentrations, it has surprisingly been found that as little as 25 ppm of a 2-alkyl-3-isothiazolone can be used to prevent growth of *Candida albicans.*

Preferably, the composition (or the product/cosmetic formulation) contains a maximum of 4000 ppm of the alkane diol, more preferably a maximum of 3000 ppm.

Preferably, the composition (or the product/cosmetic formulation) contains 1ppm to 300 ppm, more preferably 5ppm to 200 ppm, more preferably 10 ppm to 100 ppm, preferably 30 to 75 ppm, more preferably from 55 ppm to 75 ppm of the 2-alkyl-3-isothiazolone. Preferably the composition comprises from 1000 ppm to 3000 ppm of the alkane diol, more preferably from 1500 ppm to 2500 ppm alkane diol. The chlorphenesin is preferably present in the composition in an amount of 30 to 4000 ppm. The low concentration of the isothiazolone would not on its own be expected to prevent growth of bacteria and fungi to a satisfactory degree, if at all. However, the additional presence of the alkane diol and/or the chlorphenesin results unexpectedly in a composition with effective biocidal properties.

The composition preferably contains no chlorinated isothiazolones.

The composition may be made by combining and preferably mixing the components. The composition may be made at room temperature (about 20 °C) or with heating, as desired.

The cosmetic formulation according to the present invention may be in the form of a cream, a lotion, a paste, a liquid, an aerosol, a shampoo, a gel, a wipe, a bar, a stick, a powder and/or granules or any other form suitable for application to the skin, including the scalp and the mucosa including the lips.

The compositions or formulations according to the present invention preferably are formulated into forms that are useful in sunscreen products. In particular, they are preferably formulated as emulsions.

The cosmetic formulations according to the present invention may additionally contain one or more conventional ingredients or additives such as a surfactant, an emulsifier, a consistency factor, a conditioner, an emollient, a skin caring ingredient, a moisturizer, a thickener, a glidant, a lubricant, a filler, a binding agent, an anti-oxidant, a preservative, an active ingredient (eg dermatologically active ingredients) and a fragrance. Examples of active ingredients include anti-inflammatories and anti-allergics. Active ingredients suited for topical applications are particularly preferred.

Suitable surfactants include, but are not limited to, alkyl sulphates (for example, sodium lauryl sulphate, ammonium lauryl sulphate, sodium cetearyl sulphate) alkyl sulphoacetates (for example sodium lauryl sulphoacetate) alkyl ether sulphates (for example sodium laureth sulphate, sodium trideceth sulphate, sodium oleth sulphate, ammonium laureth sulphate), alkyl ether sulphosuccinates (for example disodium laureth sulphosuccinate) alkyl glycosides (for example decyl glucoside, lauryl glucoside), alkyl isethionates, amphoterics (for example cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium salts of the aforementioned amphoterics, capryl/capramidopropyl betaine, undecylenamidopropyl betaine, lauramidopropyl betaine) and fatty alcohol polyglycol ethers.

Suitable emulsifiers include, but are not limited to, salts of fatty acids (for example sodium stearate or sodium palmitate), organic soaps (for example, mono-, di- or triethanolaminoleate), sulphates or sulphonated compounds (for example sodium lauryl sulphate or sodium cetyl sulphonate), saponines, lamepones, quaternary ammonium salts, fatty alcohols, fatty acid esters derived from saturated or unsaturated fatty acids, polyoxyethylenesters or polyoxyethylenethers of fatty acids, polymers of ethylene oxide and propylene oxide or propylene glycol, phosphatides, gelatine, casein alkylamidobetaines, alkyl betaines, alkyl amphoglycinates, alkyl phosphates, alkylpolyoxyethylene phosphates or the corresponding acids and silicone derivatives (for example alkyl dimethiconecopolyol).

Suitable consistency factors include, but are not limited to, fatty alcohols or their mixtures with fatty acid esters, (for example, acetylated lanolin alcohol), aluminium stearates, carbomer, cetyl alcohol, glyceryl oleate, glyceryl stearate, glyceryl stearate, PEG 100 stearate, magnesium stearate, magnesium sulphate, oleic acid, stearic acid, stearyl alcohol, myristyl myristate, isopropyl palmitate and beeswax and synthetic equivalents thereof.

Suitable conditioners include, but are not limited to, alkylamido ammonium lactate, cetrimonium chloride, distearoylethyl hydroxyethylammonium methosulphate, cetearyl alcohol, cetyl dimethicone, cetyl ricinoleate, dimethicone, laureth-23, laureth-4, polydecene, retinyl palmitate, quaternised protein hydrolysates, quaternised cellulose and starch derivatives, quaternised copolymers of acrylic or methacrylic acid or salts and quaternised silicone derivatives.

Suitable emollients include, but are not limited to, cetearyl isononanoate, cetearyl octanoate, decyl oleate, isooctyl stearate, coco caprylate/caprate, ethylhexyl hydroxystearate, ethylhexyl isononanoate, isopropyl isostearate, isopropyl myristate, oleyl oleate, hexyl laurate, paraffinum liquidum, PEG-75 lanolin, PEG-7 glyceryl cocoate, petrolatum, ozokerite, cyclomethicone, dimethicone, dimethicone copolyol, dicaprylyl ether, butyrospermum parkii, buxus chinensis, canola, carnauba cera, copernicia cerifera, oenothera biennis, elaeis guineensis, prunus dulcis, squalane, zea mays, glycine soja, lanolin, hydrogenated castor oil, hydrogenated coconut oil, hydrogenated polyisobutene, sucrose cocoate, stearoxy dimethicone, lanolin alcohol and isohexadecane.

Suitable skin caring ingredients include, but are not limited to, plant extracts, bisabolol, anti-inflammatory agents, urea, allantoin, panthenol and panthenol derivatives, phytantriol, vitamins A, B5, E, C and D, ceramides of animal or plant origin and lecithins.

Suitable moisturizers include, but are not limited to, butylene glycol, cetyl alcohol, dimethicone, dimyristyl tartrate, glucose, glycereth-26, glycerin, glyceryl stearate, hydrolyzed milk protein, lactic acid, lactose and other sugars, laureth-8, lecithin, octoxyglycerin, PEG-12, PEG-135, PEG-150, PEG-20, PEG-8, phytantriol, polyquaternium-39, PPG-20 methyl glucose ether, propylene glycol, sodium hyaluronate, sodium lactate, sodium PCA, sorbitol, succinoglycan, synthetic beeswax, tri-C$_{14-15}$ alkyl citrate and starch.

Suitable thickeners include, but are not limited to, acrylate/steareth-20 methacrylate copolymers, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl silylate, xanthan gum and hydrogenated butylenes/ethylene/styrene copolymer.

Suitable lubricants include, but are not limited to, adipic acid, fumaric acid and its salts, benzoic acid and its salts, glycerine triacetate, sodium or magnesium lauryl sulphate, magnesium stearate, solid polyethylenglycol, polyvinylpyrrolidone, boric acid, monolaurate or - palmitate, myristyl alcohol, cetyl alcohol, cetylstearyl alcohol, talcum, calcium or magnesium salts of higher fatty acids, mono-, di-or triglycerides of higher fatty acids and polytetrafluorethylene.

Suitable anti-oxidants include, but are not limited to, sulphites (for example, sodium sulphite, tocopherol or derivates thereof), ascorbic acid or derivates thereof, citric acid, propyl gallate, chitosan glycolate, cysteine, N-acetyl cysteine plus zinc sulphate, thiosulphates (for example sodium thiosulphate) and polyphenoles.

## Examples

The following Examples (see Tables I to VI) show the unexpected synergy of the non-halogenated 2-alkyl-3-isothiazolone and the alkane diol in the preservative composition of the invention.

For this purpose, aqueous mixtures were produced with various concentrations of 2-methylisothiazolin-3-one (MIT) and caprylyl glycol (CAG) and the activity of these mixtures were tested on the micro-organisms listed in Table VI.

In addition to the active component(s) and water, the aqueous mixtures contained a nutrient medium, namely a Müller-Hinton broth (Manufacturer: Oxoid; product code CM405). The incubation time was 72h at 25°C (bacterial and yeast) or 7 days at 25°C (mould). Each sample was incubated on an incubation shaker at 105 rpm.

Tables I to V show the concentrations of MIT and CAG used in each Example. The tables also show whether growth of the micro-organism took place (symbol "+") or not (symbol "-").

Tables I to V also show the minimum inhibition concentrations (MIC). For example, in Table I, an MIC value of 25 ppm is found when MIT is used alone and an MIC value of 4000 ppm when CAG is used alone. In contrast, the MIC value of a mixture of MIT and CAG is clearly lower, i.e., when they are combined, MIT and CAG act synergistically.

### Table I

| MIC values for Pseudomonas aeruginosa after an incubation time of 72h | | | | | | |
|---|---|---|---|---|---|---|
| MIT | CAG concentration (ppm) | | | | | |
| concentration (ppm) | 0 | 500 | 1000 | 2000 | 3000 | 4000 |
| 75 | - | - | - | - | - | - |
| 50 | - | - | - | - | - | - |
| 37.5 | - | - | - | - | - | - |
| 25 | - | - | - | - | - | - |
| 10 | + | - | - | - | - | - |
| 0 | + | + | + | + | + | - |

### Table II

| MIC values for *Staphylococcus* aureus after an incubation time of 72h | | | | | | |
|---|---|---|---|---|---|---|
| MIT concentration (ppm) | CAG concentration (ppm) | | | | | |
| | 0 | 500 | 1000 | 2000 | 3000 | 4000 |
| 75 | - | - | - | - | - | - |
| 50 | - | - | - | - | - | - |
| 37.5 | - | - | - | - | - | - |
| 25 | - | - | - | - | - | - |
| 10 | + | - | - | - | - | - |
| 0 | + | + | - | - | - | - |

### Table III

| MIC values for *Enterococcus faecalis* after an incubation time of 72h | | | | | | |
|---|---|---|---|---|---|---|
| MIT concentration (ppm) | CAG concentration (ppm) | | | | | |
| | 0 | 500 | 1000 | 2000 | 3000 | 4000 |
| 75 | - | - | - | - | - | - |
| 50 | + | - | - | - | - | - |
| 37.5 | + | + | + | - | - | - |
| 25 | + | + | + | - | - | - |
| 10 | + | + | + | - | - | - |

(continued)

**MIC values for *Enterococcus faecalis* after an incubation time of 72h**

| MIT concentration (ppm) | CAG concentration (ppm) | | | | | |
|:---:|:---:|:---:|:---:|:---:|:---:|:---:|
| | 0 | 500 | 1000 | 2000 | 3000 | 4000 |
| 0 | + | + | + | - | - | - |

**Table IV**

**MIC values for *Aspergillus niger* after an incubation time of 5 days**

| MIT concentration (ppm) | CAG concentration (ppm) | | | | | |
|:---:|:---:|:---:|:---:|:---:|:---:|:---:|
| | 0 | 500 | 1000 | 2000 | 3000 | 4000 |
| 300 | - | - | - | - | - | - |
| 200 | + | - | - | - | - | - |
| 100 | + | + | - | - | - | - |
| 75 | + | + | - | - | - | - |
| 50 | + | + | - | - | - | - |
| 37.5 | + | + | + | - | - | - |
| 25 | + | + | + | - | - | - |
| 10 | + | + | + | - | - | - |
| 0 | + | + | + | - | - | - |

**Table V**

**MIC values for *Candida albicans* after an incubation time of 72h**

| MIT concentration (ppm) | CAG concentration (ppm) | | | | | |
|:---:|:---:|:---:|:---:|:---:|:---:|:---:|
| | 0 | 500 | 1000 | 2000 | 3000 | 4000 |
| 200 | - | - | - | - | - | - |
| 100 | + | - | - | - | - | - |
| 75 | + | - | - | - | - | - |
| 50 | + | + | - | - | - | - |
| 37.5 | + | + | + | - | - | - |
| 25 | + | + | + | - | - | - |
| 10 | + | + | + | + | - | - |
| 0 | + | + | + | + | - | - |

The synergy index is calculated according to the method of F.C. Kull et al., Applied Microbiology, Vol. 9 (1961), p. 538. Here the synergy index is calculated using the following formula:

$$\text{Synergy index} = Q_a/Q_A + Q_b/Q_B$$

When this formula is used on the biocide system tested here, the quantities in the formula have the following meaning:

$Q_a$ = MIC of MIT in the preservative mixture of MIT and CAG
$Q_A$ = MIC of MIT as sole ingredient
$Q_b$ = MIC of CAG in the preservative mixture of MIT and CAG
$Q_B$ = MIC of CAG as sole ingredient

When the synergy index exhibits a value greater than 1, this means that there is an antagonism. When the synergy index has the value 1, this means that there is an addition of the activity of the two ingredients. When the synergy index has a value of less than 1, this means that a synergy of the two ingredients exists.

**Table VI**

**Synergy indices calculated from data in Tables I - V**

| Organism | Strain | MIC values (ppm) | | | | Synergy index |
|---|---|---|---|---|---|---|
| | | $Q_A$ (MIT) | $Q_B$ (CAG) | $Q_a$ (MIT) | $Q_b$ (CAG) | |
| *Pseudomonas aeruginosa* | ATCC 9027 | 25 | 4000 | 10 | 500 | 0.53 |
| *Staphylococcus aureus* | ATCC 6538 | 25 | 1000 | 10 | 500 | 0.90 |
| *Enterococcus faecalis* | NCIMB 12756 | 75 | 2000 | 50 | 500 | 0.92 |
| *Aspergillus niger* | IMI 149007 | 300 | 2000 | 50 | 1000 | 0.67 |
| *Candida albicans* | NCPF 3179 | 200 | 3000 | 75 | 500 | 0.55 |

**Test on Synergistic effect of Chlorphenesin**

A test was also carried out to assess if chlorphenesin exhibited a synergistic effect when combined with a composition comprising both MIT and CAG. The test was carried out in a similar manner as above.

For this purpose, aqueous mixtures were produced containing various concentrations of both (i) a composition comprising MIT and CAG in a 1:30 weight ratio and (ii) chlorphenesin and the activity of these mixtures were tested on the micro-organisms listed in Table VII.

In addition to the active component(s) and water, the aqueous mixtures contained a nutrient medium, namely a Müller-Hinton broth (Manufacturer: Oxoid; product code CM405). The incubation time was 72h at 25°C (bacterial and yeast) or 7 days at 25°C (mould). Each sample was incubated on an incubation shaker at 105 rpm.

In an analogous manner as for the Examples above, for each micro-organism, the MIC was determined for a composition comprising MIT and CAG in a weight ratio of 1:30 (i) in the absence of chlorphenesin ($Q_{A(MIC+CAG)}$) and (ii) in the presence of chlorphenesin ($Q_{a(MIC+CAG)}$).

The MIC of chlorphenesin was likewise determined (i) for chlorphenesin as a sole ingredient ($Q_{B(CP)}$) and (ii) for chlorphenesin in the presence of a composition comprising MIT and CAG in a weight ratio of 1:30 ($Q_{b(CP)}$).

The synergy index (SI) for each micro-organism was calculated as follows:

$$\text{Synergy index} = Q_{a(MIC+CAG)}/Q_{A(MIC+CAG)} + Q_{b(CP)}/Q_{B(CP)}.$$

The results are as shown in Table VII:

**Table VII**

| | $Q_{A(MIT/CAG)}$ | $Q_{B(CP)}$ | $Q_{a(MIT/CAG)}$ | $Q_{b(CP)}$ | SI |
|---|---|---|---|---|---|
| *E. Coli* | 620 | 1500 | 310 | 200 | 0.70 |
| *Staphylococcus aureus* | 620 | 3000 | 310 | 1000 | 0.83 |
| *Candida albicans* | 930 | 2000 | 620 | 400 | 0.87 |

One can see from this table that chlorphenesin and a composition of MIT and CAG acted synergistically for *E. Coli, St. aureus* and *Candida albicans*.

Analogous experiments were carried out as above for $C_{10}$ to $C_{12}$ alkane diols. It was surprising found that $C_{10}$ to $C_{12}$ alkane diols exhibited greater biocidal efficacy than $C_8$ alkane diols when in combination with MIT.

Tables VIIIA to VIIID - Efficacy of $C_5$ alkane diols vs. $C_8$ alkane diols.

**Table VIIIA. *Pseudomonas aeruginosa***

| | 0% | 0.2% | 0.4% | 0.6% | 0.8% | 1.0% |
|---|---|---|---|---|---|---|
| 1,2 Pentane diol | + | + | + | + | + | + |
| 1,2-Octane diol | + | + | - | - | - | - |

**Table VIIIB. *Staphylococcus Aureus***

|  | 0% | 0.2% | 0.4% | 0.6% | 0.8% | 1.0% |
|---|---|---|---|---|---|---|
| 1,2 Pentane diol | + | + | + | + | + | + |
| 1,2-Octane diol | + | - | - | - | - | - |

**Table VIIIC. *Candida Albicans***

|  | 0% | 0.2% | 0.4% | 0.6% | 0.8% | 1.0% |
|---|---|---|---|---|---|---|
| 1,2 Pentane diol | + | + | + | + | + | + |
| 1,2-Octane diol | + | + | - | - | - | - |

**Table VIIID. *Aspergillus Niger***

|  | 0% | 0.2% | 0.4% | 0.6% | 0.8% | 1.0% |
|---|---|---|---|---|---|---|
| **1,2 Pentane diol** | + | + | + | + | + | + |
| **1,2-Octane diol** | + | - | - | - | - | - |
| **+ = no growth inhibition** <br> **- = growth inhibition** | | | | | | |

It has been found by the inventors that alkane diols exhibit different anti-microbial activities. Tables VIIIA to VIIID above illustrate the results of tests of the efficacy of octane diol compared to pentane diol on various organisms. These tests were carried out in a similar manner to those described above. The percentages at the top line of each table indicate the amount of diol used by weight in the preservative mixture.

**Test on a model formulated skin cream**

**Preservative Efficacy Tests (PETs)**

Typical formulations of the active components were subjected to a PET using a model formulated skin cream of the following composition:

**Skin Cream**

| Material | INCI | % |
|---|---|---|
| Water Deionised | Aqua to | 100 |
| Polawax GP200 |  | 8.0 |
| Glycerine BP | Glycerin | 5.0 |
| Propylene Glycol BP | Propylene Glycol | 5.0 |
| Fractionated Coconut Oil | Caprylic/Capric Triglyceride | 4.0 |
| Sweet Almond Oil | Prunus Dulcis | 4.0 |
| Light Liquid Paraffinum | Paraffinum Liquidum | 3.0 |
| RD Coconut Oil | Cocus Nucifera Oil | 2.0 |
| Lasermul 92AE | Glyceryl Stearate SE | 2.0 |
| Aloe Vera x 10 | Aloe Barbadensis | 1.0 |
| Crodamol SS | Cetyl Esters | 1.0 |
| Cucumber Extract | Cucumis Sativus | 0.5 |

(continued)

| Material | INCI | % |
|---|---|---|
| Ground Ivy Extract | Glechoma Hederacea | 0.5 |
| Ginseng Extract | Panax Ginseng | 0.5 |
| Fragrance | Parfum | 0.5 |
| Vitamin E Acetate | Tocopheryl Acetate | 0.2 |
| Vitamin A Palmitate | Retinyl Palmitate | 0.1 |
| FD&C Blue No 1 | CI 42090 | 0.0003 |
| FD&C Yellow No 5 | CI 19140 | 0.0005 |

The test procedure, based on the British Pharmacopoeia 2004 (Appendix XVIC), was as follows. Aliquots of the preserved skin cream were inoculated using single bacteria or fungi. The inoculated aliquots were incubated at 25°C and Ig samples removed after 0, 2, 7, 14 and 28 days. Total viable counts (TVC) to determine surviving bacteria or fungi were performed after preservative inactivation using Letheen Broth. Where applicable serial dilutions were carried out using quarter strength Ringers Solution. Bacteria were plated onto Tryptone Soya Agar and incubated at 33°C for 72 hours, whilst fungi were plated onto Sabouraud Dextrose Agar and incubated at 23°C for 120 hours.

The pass criteria for the preservatives were as follows:

**Bacteria**

Reduction in TVC of $10^2$ bacteria (with respect to NC at Time 0) within 48 hours of the challenge and $10^3$ bacteria after 7 days with no increase thereafter.

**Table IX PET Results for Microcare® MTG (Mixture of MIT and CAG)**

| Organism | Preservative % | 0 days | 2 days | 7 days | 14 days | 28 days |
|---|---|---|---|---|---|---|
| A. niger IMI 149007 | 0.20 | $2.0 \times 10^5$ | $1.8 \times 10^5$ | $6.0 \times 10^4$ | $4.0 \times 10^4$ | $1.0 \times 10^4$ |
| | 0.30 | $1.6 \times 10^5$ | $6.2 \times 10^4$ | $1.2 \times 10^4$ | $2.0 \times 10^4$ | $1.6 \times 10^3$ |
| | 0.40 | $2.2 \times 10^5$ | $1.6 \times 10^4$ | $4.0 \times 10^3$ | $1.0 \times 10^3$ | $2.0 \times 10^1$ |
| | 0.50 | $2.6 \times 10^5$ | $2.6 \times 10^3$ | $2.0 \times 10^2$ | <20 | <20 |
| C. albicans NCPF 3197 | 0.20 | $1.0 \times 10^6$ | $8.0 \times 10^5$ | $1.0 \times 10^5$ | $2.0 \times 10^5$ | $1.2 \times 10^5$ |
| | 0.30 | $4.0 \times 10^5$ | $1.2 \times 10^5$ | $2.0 \times 10^2$ | <20 | <20 |
| | 0.40 | $1.0 \times 10^6$ | $6.0 \times 10^5$ | <20 | <20 | <20 |
| | 0.50 | $4.0 \times 10^5$ | $1.0 \times 10^5$ | <20 | <20 | <20 |
| Ps. Aeruginosa ATCC 9027 | 0.20 | $4.0 \times 10^6$ | $5.0 \times 10^5$ | <20 | <20 | <20 |
| | 0.30 | $4.0 \times 10^6$ | $2.6 \times 10^5$ | <20 | <20 | <20 |
| | 0.40 | $1.8 \times 10^7$ | $1.1 \times 10^5$ | <20 | <20 | <20 |
| | 0.50 | $1.2 \times 10^7$ | $2.4 \times 10^4$ | <20 | <20 | <20 |
| S. aereus ATCC 6538 | 0.20 | $3.2 \times 10^7$ | $1.0 \times 10^7$ | <20 | <20 | <20 |
| | 0.30 | $2.8 \times 10^7$ | $9.4 \times 10^6$ | <20 | <20 | <20 |
| | 0.40 | $4.8 \times 10^7$ | $7.2 \times 10^6$ | <20 | <20 | <20 |
| | 0.50 | $5.6 \times 10^7$ | $1.6 \times 10^6$ | <20 | <20 | <20 |

(continued)

| Organism | Preservative % | 0 days | 2 days | 7 days | 14 days | 28 days |
|---|---|---|---|---|---|---|
| B. cepacia NCIMB 9085 | 0.20 | $8.0 \times 10^6$ | $1.8 \times 10^6$ | <20 | <20 | <20 |
| | 0.30 | $4.0 \times 10^6$ | $6.2 \times 10^5$ | <20 | <20 | <20 |
| | 0.40 | $1.0 \times 10^7$ | $6.6 \times 10^4$ | <20 | <20 | <20 |
| | 0.50 | $1.4 \times 10^7$ | $5.0 \times 10^3$ | <20 | <20 | <20 |

**Table X PET Results for Microcare® MTC (Mixture of MIT and Chlorphenesin)**

| Organism | WR218 (%) | 0 hours | 48 hours | 7 days | 14 days | 28 days |
|---|---|---|---|---|---|---|
| A. niger IMI 14907 | 0.20 | $4.0 \times 10^5$ | $2.0 \times 10^5$ | $2.0 \times 10^5$ | $2.0 \times 10^5$ | $6.0 \times 10^4$ |
| | 0.60 | $4.0 \times 10^5$ | $4.0 \times 10^5$ | $8.0 \times 10^4$ | $2.0 \times 10^4$ | $2.0 \times 10^4$ |
| | 1.00 | $2.0 \times 10^5$ | $6.0 \times 10^4$ | $4.0 \times 10^4$ | $4.0 \times 10^2$ | <20 |
| C. albicans NCPF 3179 | 0.20 | $5.0 \times 10^5$ | $1.3 \times 10^6$ | $2.0 \times 10^6$ | $8.6 \times 10^5$ | $6.0 \times 10^5$ |
| | 0.60 | $7.4 \times 10^5$ | $2.6 \times 10^5$ | $4.4 \times 10^4$ | $2.0 \times 10^4$ | $4.0 \times 10^2$ |
| | 1.00 | $6.6 \times 10^5$ | $2.6 \times 10^5$ | $2.0 \times 10^2$ | <20 | <20 |
| Ps. aeruginosa ATCC 9027 | 0.20 | $1.4 \times 10^7$ | $2.2 \times 10^6$ | <20 | <20 | <20 |
| | 0.60 | $8.0 \times 10^6$ | $5.2 \times 10^5$ | <20 | <20 | <20 |
| | 1.00 | $6.2 \times 10^6$ | $1.6 \times 10^5$ | <20 | <20 | <20 |
| S. aereus ATCC 6538 | 0.20 | $1.8 \times 10^7$ | $1.8 \times 10^7$ | $2.2 \times 10^5$ | <20 | <20 |
| | 0.60 | $3.0 \times 10^7$ | $2.6 \times 10^7$ | $2.8 \times 10^5$ | <20 | <20 |
| | 1.00 | $1.6 \times 10^7$ | $1.2 \times 10^7$ | $4.6 \times 10^4$ | <20 | <20 |
| B. cepacia NCIMB 9085 | 0.20 | $6.2 \times 10^6$ | $8.0 \times 10^5$ | $1.6 \times 10^4$ | <20 | <20 |
| | 0.60 | $6.2 \times 10^6$ | $1.2 \times 10^6$ | $2.0 \times 10^1$ | <20 | <20 |
| | 1.00 | $6.2 \times 10^6$ | $2.0 \times 10^6$ | <20 | <20 | <20 |

**Claims**

1. A microbiocidal composition comprising: a non-halogenated 2-alkyl-3-isothiazolone and an alkane diol, wherein the alkyl group in the non-halogenated 2-alkyl-3-isothiazolone comprises 1 to 4 carbon atoms, the alkane diol comprises from 9 to 12 carbon atoms, and the hydroxyl groups of the alkane diol are vicinally substituted.

2. A microbiocidal composition according to claim 1, wherein the alkane diol is 1,2-decanediol.

3. A microbiocidal composition according to claim 1 or 2, wherein the non-halogenated 2-alkyl-3- isothiazolone is 2-methyl-3-isothiazolone.

4. A microbiocidal composition according any one of the preceding claims comprising the 2-alkyl-3-isothiazolone and the alkane diol in a ratio range (wt.%) of from 1:10 to 1:30.

5. A microbiocidal composition according to any one of the preceding claims, wherein the composition further comprises a water-soluble organic solvent.

6. A microbiocidal composition according to claim 5, wherein the organic solvent comprises one or more of ethylene glycol, diethylene glycol, polyethylene glycol, propylene glycol, dipropylene glycol, polypropylene glycol, phenoxyethanol and benzyl alcohol.

7. A cosmetic formulation comprising a microbiocidal composition as defined in any one of claims 1 to 7.

8. A cosmetic formulation as claimed in claim 7, wherein the formulation contains from 1 ppm to 100 ppm of the 2-alkyl- 3-isothiazolone, and from 300 ppm to 4000 ppm of the alkane diol.

9. A cosmetic formulation according to claim 7 or 8, wherein the cosmetic formulation is selected from a moisturising cream, a sunscreen, a liquid soap, a conditioner and a shampoo.

10. Use of the microbiocidal composition according to any one of claims 1 to 6 in a cosmetic formulation.

11. Use of the microbiocidal composition according to claim 10 in moisturising cream, sunscreen, liquid soap, conditioner or shampoo.

12. Use of the microbiocidal composition as defined in any one of claims 1 to 6 in product selected from: a paint, a plaster composition, a lignin sulphonate, a whitewash, an adhesive, a photochemical composition, a product containing casein, a product containing starch, an asphalt emulsion, a surfactant solution, a fuel, a cleaning agent, a water system, a polymer dispersion or a lubricant.

13. Use according to claim 12, wherein the product contains from 1 ppm to 300 ppm of the 2-alkyl-3-isothiazolone, and from 300 ppm to 4000 ppm of the alkane diol.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **F.C. KULL et al.** *Applied Microbiology,* 1961, vol. 9, 538